# EUROPÄISCHE PATENTANMELDUNG

(11) **EP 0 765 867 A1**
(43) Veröffentlichungstag der Anmeldung: **02.04.1997**
(21) Anmeldenummer: 95115240.4
(22) Anmeldetag: 27.09.1995
(51) Int. Cl.: C07C 279/22, A61K 31/155

(54) **Substituierte Benzoylguanidine, Verfahren zu ihrer Herstellung, ihre Verwendung als Antiarrhytmika oder Diagnostikum sowie sie enthaltendes Medikament**

(71) Anmelder: HOECHST AKTIENGESELLSCHAFT, 65929 Frankfurt am Main (DE)
(72) Erfinder: Die Erfindernennung liegt noch nicht vor

(57) **Zusammenfassung**

Verbindungen der Formel I 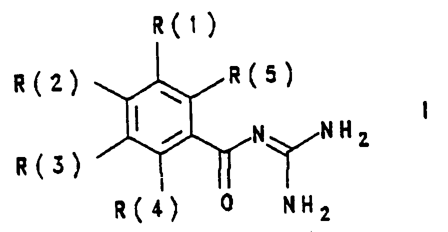 worin
mindestens einer der Substituenten R(1), R(2) und R(3)
R(6)-C(OH)₂- bedeutet, mit
R(6) Perfluoralkyl mit 1, 2 oder 3-C-Atomen, das geradkettig oder verzweigt ist;
sowie ihre pharmazeutische verträglichen Salze werden erhalten durch Umsetzung einer Verbindung II 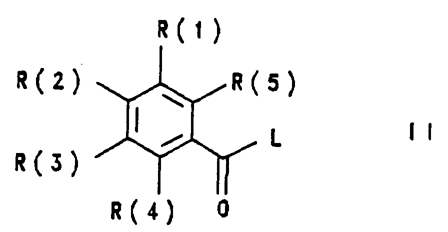 mit Guanidin, worin R(1) bis R(5) die angegebene Bedeutung besitzen und L für eine leicht neclepohil substituierbare leaving group steht. Verbindungen I sind hervorragende Herz-Kreislauf-Therapeutika.

## Beschreibung

Die Erfindung betrifft Benzoylguanidine der Formel I 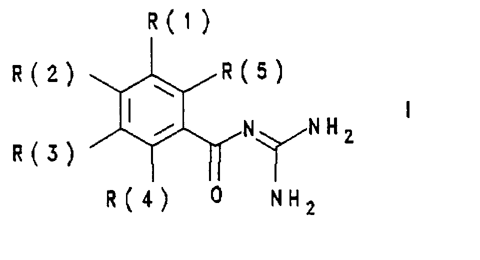 worin bedeuten:
mindestens einer der Substituenten R(1), R(2) und R(3)
R(6)-C(OH)₂-;
- R(6): Perfluoralkyl mit 1, 2 oder 3-C-Atomen, das geradkettig oder verzweigt ist;
und die übrigen Substituenten R(1), R(2) und R(3)
unabhängig voneinander Wasserstoff, OH, F, Cl, Br, I, Alkyl mit 1, 2, 3, 4, 5 oder 6 C-Atomen, Cycloalkyl mit 3, 4, 5 oder 6 C-Atomen, Alkoxy mit 1, 2, 3 oder 4 C-Atomen oder Phenoxy,
das unsubstituiert ist oder substituiert ist mit 1 - 3 Substituenten ausgewählt auf der Gruppe bestehend aus F, Cl, Methyl und Methoxy;
oder
die übrigen Substituenten R(1), R(2) und R(3)
unabhängig voneinander Alkyl-SOₓ, -CR(7)=CR(8)R(9) oder -C≡CR(9);
- x: Null, 1 oder 2;
- R(7): Wasserstoff oder Methyl;
- R(8) und R(9): unabhängig voneinander Wasserstoff, Alkyl mit 1, 2, 3 oder 4 C-Atomen, Cycloalkyl mit 3, 4, 5, 6, 7 oder 8 C-Atomen oder Phenyl,
das unsubstituiert ist oder substituiert ist mit 1 - 3 Substituenten ausgewählt auf der Gruppe bestehend aus F, Cl, CF₃, Methyl und Methoxy;
oder
die übrigen Substituenten R(1), R(2) und R(3)
unabhängig voneinander Phenyl, C₆H₅-(C₁-C₄)-Alkyl, Naphthyl, Biphenylyl, Chinolinyl, Isochinolinyl oder Imidazolyl,
wobei Chinolinyl, Isochinolinyl oder Imidazolyl über C oder N gebunden sind, und wobei Phenyl, C₆H₅-(C₁-C₄)-Alkyl, Naphthyl, Biphenylyl, Chinolinyl, Isochinolinyl und Imidazolyl unsubstituiert sind oder substituiert sind mit 1 - 3 Substituenten ausgewählt aus der Gruppe bestehend aus F, Cl, CF₃, CH₃, Methoxy, Hydroxy, Amino, Methylamino und Dimethylamino;
oder
die übrigen Substituenten R(1), R(2) und R(3)
unabhängig voneinander SR(10), -OR(10), -CR(10)R(11)R(12);
- R(10): -C_{f}H_{2f}-(C₃-C₈)-Cycloalkyl, Chinolinyl, Isochinolinyl, Pyridinyl, Imidazolyl oder Phenyl,
wobei die aromatischen Systeme Chinolinyl, Isochinolinyl, Pyridinyl, Imidazolyl und Phenyl unsubstituiert sind oder substituiert sind mit 1 - 3 Substituenten ausgewählt aus der gruppe bestehend aus F, Cl, CF₃, CH₃, Methoxy, Hydroxy, Amino, Methylamino und Dimethylamino;
- f: Null, 1 oder 2;
- R(11) und R(12): unabhängig voneinander wie R(10) definiert, Wasserstoff oder Alkyl mit 1, 2, 3 oder 4 C-Atomen;
- R(4) und R(5): unabhängig voneinander Wasserstoff, Alkyl mit 1, 2 oder 3-C-Atomen, F, Cl, Br, I, CN, OR(13), NR(14)R(15), -(CH₂)ₙ-(CF₂)ₒ-CF₃;
- R(13), R(14) und R(15): unabhängig voneinander Wasserstoff oder Alkyl mit 1, 2, 3 oder 4 C-Atomen;
- n: Null oder 1;
- o: Null, 1 oder 2;
sowie deren pharmakologisch akzeptable Salze.

Bevorzugt sind Verbindungen der Formel I, in denen bedeuten:
mindestens einer der Substituenten R(1), R(2) und R(3)
CF₃-C(OH)₂-;
und die übrigen Substituenten R(1), R(2) und R(3)
unabhängig voneinander Wasserstoff, F, Cl, Alkyl mit 1, 2, 3, 4, 5 oder 6 C-Atomen, Cycloalkyl mit 3, 4, 5 oder 6 C-Atomen, Alkoxy mit 1, 2, 3 oder 4 C-Atomen oder Phenoxy,
das unsubstituiert ist oder substituiert ist mit 1 - 3 Substituenten ausgewählt auf der Gruppe bestehend aus F, Cl, Methyl und Methoxy;
oder
die übrigen Substituenten R(1), R(2) und R(3)
unabhängig voneinander Alkyl-SOₓ, -CR(7)=CR(8)R(9) oder -C≡CR(9);
- x: Null, 1 oder 2;
- R(7): Wasserstoff oder Methyl;
- R(8) und R(9): unabhängig voneinander Wasserstoff, Alkyl mit 1, 2, 3 oder 4 C-Atomen, Cycloalkyl mit 3, 4, 5, 6, 7 oder 8 C-Atomen oder Phenyl,
das unsubstituiert ist oder substituiert ist mit 1 - 3 Substituenten ausgewählt auf der Gruppe bestehend aus F, Cl, CF₃, Methyl und Methoxy;
oder
die übrigen Substituenten R(1), R(2) und R(3)
unabhängig voneinander Phenyl, C₆H₅-(C₁-C₂)-Alkyl, Naphthyl, Biphenylyl, Chinolinyl, Isochinolinyl oder Imidazolyl,
wobei Chinolinyl, Isochinolinyl oder Imidazolyl über C oder N gebunden sind, und wobei Phenyl, C₆H₅-(C₁-C₄)-Alkyl, Naphthyl, Biphenylyl, Chinolinyl, Isochinolinyl und Imidazolyl unsubstituiert sind oder substituiert sind mit 1 - 3 Substituenten ausgewählt aus der Gruppe bestehend aus F, Cl, CF₃, CH₃, Methoxy, Hydroxy, Amino, Methylamino und Dimethylamino;
oder
die übrigen Substituenten R(1), R(2) und R(3)
unabhängig voneinander SR(10), -OR(10), -CR(10)R(11)R(12);
- R(10): -C_{f}H_{2f}-(C₃-C₈)-Cycloalkyl, Chinolinyl, Isochinolinyl, Pyridinyl, Imidazolyl oder Phenyl,
wobei die aromatischen Systeme Chinolinyl, Isochinolinyl, Pyridinyl, Imidazolyl und Phenyl unsubstituiert sind oder substituiert sind mit 1 - 3 Substituenten ausgewählt aus der gruppe bestehend aus F, Cl, CF₃, CH₃, Methoxy, Hydroxy, Amino, Methylamino und Dimethylamino;
- f: Null oder 1;
- R(11) und R(12): unabhängig voneinander wie R(10) definiert, Wasserstoff oder Alkyl mit 1, 2, 3 oder 4 C-Atomen;
- R(4) und R(5): unabhängig voneinander Wasserstoff, Alkyl mit 1, 2 oder 3-C-Atomen, F, Cl, OH, Methoxy, NH₂, -(CF₂)ₒ-CF₃;
- o: Null oder 1;
sowie deren pharmakologisch akzeptable Salze.

Ganz besonders bevorzugt sind Verbindungen der Formel I, in denen bedeuten:
- R(1): CF₃-C(OH)₂-;
- R(2) und R(3): unabhängig voneinander Wasserstoff, F, Cl, Alkyl mit 1, 2, 3 oder 4 C-Atomen, Cycloalkyl mit 3, 4, 5 oder 6 C-Atomen, Alkoxy mit 1, 2, 3 oder 4 C-Atomen oder Phenoxy,
das unsubstituiert ist oder substituiert ist mit 1 - 3 Substituenten ausgewählt auf der Gruppe bestehend aus F, Cl, Methyl und Methoxy;
oder
- R(2) und R(3): unabhängig voneinander -CR(7)=CR(8)R(9) oder -C≡CR(9);
- R(7): Wasserstoff oder Methyl;
- R(8) und R(9): unabhängig voneinander Wasserstoff, Alkyl mit 1, 2, 3 oder 4 C-Atomen, Cycloalkyl mit 3, 4, 5, 6, 7 oder 8 C-Atomen oder Phenyl,
das unsubstituiert ist oder substituiert ist mit 1 - 3 Substituenten ausgewählt auf der Gruppe bestehend aus F, Cl, CF₃, Methyl und Methoxy;
oder
- R(2) und R(3): unabhängig voneinander Phenyl, C₆H₅-(C₁-C₂)-Alkyl, Naphthyl, Biphenylyl, Chinolinyl, Isochinolinyl oder Imidazolyl,
wobei Chinolinyl, Isochinolinyl oder Imidazolyl über C oder N gebunden sind, und wobei Phenyl, C₆H₅-(C₁-C₄)-Alkyl, Naphthyl, Biphenylyl, Chinolinyl, Isochinolinyl und Imidazolyl unsubstituiert sind oder substituiert sind mit 1 - 3 Substituenten ausgewählt aus der Gruppe bestehend aus F, Cl, CF₃, CH₃, Methoxy, Hydroxy, Amino, Methylamino und Dimethylamino;
oder
- R(2) und R(3): unabhängig voneinander SR(10), -OR(10), -CR(10)R(11)R(12);
- R(10): -C_{f}H_{2f}-(C₃-C₈)-Cycloalkyl, Chinolinyl, Isochinolinyl, Pyridinyl, Imidazolyl oder Phenyl,
wobei die aromatischen Systeme Chinolinyl, Isochinolinyl, Pyridinyl, Imidazolyl und Phenyl unsubstituiert sind oder substituiert sind mit 1 - 3 Substituenten ausgewählt aus der gruppe bestehend aus F, Cl, CF₃, CH₃, Methoxy, Hydroxy, Amino, Methylamino und Dimethylamino;
- f: Null oder 1;
- R(11) und R(12): unabhängig voneinander wie R(10) definiert, Wasserstoff oder Alkyl mit 1, 2, 3 oder 4 C-Atomen;
- R(4) und R(5): unabhängig voneinander Wasserstoff, Alkyl mit 1, 2 oder 3-C-Atomen, F, Cl, OCH₃, -CF₃;
sowie deren pharmakologisch akzeptable Salze.

Die bezeichneten Alkylreste können sowohl geradkettig wie verzweigt vorliegen.

Die Erfindung betrifft weiterhin ein Verfahren zur Herstellung der Verbindung I, dadurch gekennzeichnet, daß man
Verbindungen der Formel II 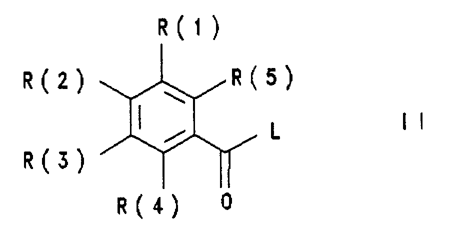 mit Guanidin umsetzt, worin R(1) bis R(5) die angegebene Bedeutung besitzen und L für eine leicht nucleophil substituierbare leaving group steht.

Die aktivierten Säurederivate der Formel II, worin L eine Alkoxy-, vorzugsweise eine Methoxygruppe, eine Phenoxygruppe, Phenylthio-, Methylthio-, 2-Pyridylthiogruppe, einen Stickstoffheterocyclus, vorzugsweise 1-Imidazolyl, bedeutet, erhält man vorteilhaft in an sich bekannter Weise aus den zugrundeliegenden Carbonsäure-chloriden (Formel II, L = Cl), die man ihrerseits wiederum in an sich bekannter Weise aus den zugrundeliegenden Carbonsäuren (Formel II, L = OH) beispielsweise mit Thionylchlorid herstellen kann.

Neben den Carbonsäurechloriden der Formel II (L = Cl) lassen sich auch weitere aktivierte Säurederivate der Formel II in an sich bekannter Weise direkt aus den zugrundeliegenden Benzoesäure- derivaten (Formel II, L = OH) herstellen, wie beispielsweise die Methylester der Formel II mit L = OCH₃ durch Behandeln mit gasförmigem HCl in Methanol, die Imidazolide der Formel II durch Behandeln mit Carbonyldiimidazol [L = 1-Imidazolyl, Staab, Angew. Chem. Int. Ed. Engl. 1, 351 -367 (1962)], die gemischten Anhydride II mit Cl-COOC₂H₅ oder Tosylchlorid in Gegenwart von Triethylamin in einem inerten Lösungsmittel. Die Aktivierung von Benzoesäuren kann auch mit Dicyclohexylcarbodiimid (DCC) oder mit O-[(Cyano(ethoxycarbonyl)methylen)amino]-1,1,3,3-tetramethyl uronium-tetrafluorborat ("TOTU") [Weiss und Krommer, Chemiker Zeitung 98, 817 (1974)] erfolgen. Eine Reihe geeigneter Methoden zur Herstellung von aktivierten Carbonsäurederivaten der Formel II sind unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel II mit Guanidin erfolgt in an sich bekannter Weise in einem protischen oder aprotischen polaren aber inerten organischen Lösungsmittel. Dabei haben sich bei der Umsetzung der Benzoesäuremethylester (II, L = OMe) mit Guanidin Methanol, Isopropanol oder THF von 20°C bis zur Siedetemperatur dieser Lösungsmittel bewährt. Bei den meisten Umsetzungen von Verbindungen II mit salzfreiem Guanidin wurde vorteilhaft in aprotischen inerten Lösungsmitteln wie THF, Dimethoxyethan, Dioxan gearbeitet. Aber auch Wasser kann unter Gebrauch einer Base wie beispielsweise NaOH als Lösungsmittel bei der Umsetzung von II mit Guanidin verwendet werden.
Wenn L = Cl bedeutet, arbeitet man vorteilhaft unter Zusatz eines Säurefängers, z. B. in Form von überschüssigen Guanidin zur Abbindung der Halogenwasserstoffsäure.

Ein Teil der zugrundeliegenden Benzoesäurederivate der Formel II ist bekannt und in der Literatur beschrieben. Die unbekannten Verbindungen der Formel II können nach literaturbekannten Methoden hergestellt werden. Die erhaltenen Benzoesäure-Derivate werden zunächst mit wäßriger Natronlauge zu den entsprechenden Bis-hydroxyverbindungen und dann nach einer der oben beschriebenen Verfahrensvarianten zu erfindungsgemäßen Verbindungen I umgesetzt.

Die Einführung einiger Substituenten gelingt durch literaturbekannte Methoden des Palladium-vermittelten cross-couplings von Arylhalogeniden bzw. Aryltriflaten mit beispielsweise Organostannanen, Organoboronsäuren oder Organoboranen oder Organokupfer- bzw. zink-verbindungen.

Die Einführung einiger Substituenten gelingt durch literaturbekannte Methoden der nucleophilen Substitution am Aromaten.

Benzoylguanidine I sind im allgemeinen schwache Basen und können Säure unter Bildung von Salzen binden. Als Säureadditionssalze kommen Salze aller pharmakologisch verträglichen Säuren infrage, beispielsweise Halogenide, insbesondere Hydrochloride, Lactate, Sulfate, Citrate, Tartrate, Acetate, Phosphate, Methylsulfonate, p-Toluolsulfonate.

Die Verbindungen I sind substituierte Acylguanidine.
Prominentester Vertreter der Acylguanidine ist das Pyrazinderivat Amilorid, das als kaliumsparendes Diuretikum in der Therapie Verwendung findet. Zahlreiche weitere Verbindungen vom Amilorid-Typ werden in der Literatur beschrieben, wie beispiels-weise Dimethylamilorid oder Ethylisopropylamilorid. 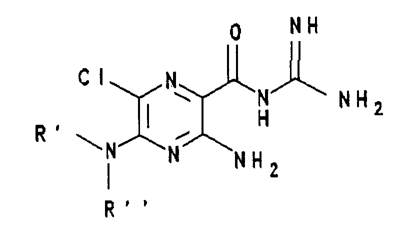
Amilorid: R',R'' = H
Dimethylamilorid: R',R'' = CH₃
Ethylisopropylamilorid: R' = C₂H₅, R'' = CH(CH₃)₂

Darüberhinaus sind Untersuchungen bekannt geworden, die auf antiarrhythmische Eigenschaften von Amilorid hinweisen (Circulation 79, 1257 - 63 (1989). Einer breiten Anwendung als Antiarrhythmikum steht jedoch entgegen, daß dieser Effekt nur schwach ausgeprägt ist und von einer blutdrucksenkenden und saluretischen Wirkung begleitet auftritt und diese Nebenwirkungen bei der Behandlung von Herz-Rhythmusstörungen unerwünscht sind.

Hinweise auf antiarrhythmische Eigenschaften des Amilorids wurden auch bei Experimenten an isolierten Tierherzen erhalten (Eur. Heart J. 9 (suppl. 1): 167 (1988) (book of abstracts). So wurde beispielsweise an Rattenherzen gefunden, daß ein künstlich ausgelöstes Kammerflimmern durch Amilorid völlig unterdrückt werden konnte. Noch potenter als Amilorid war in diesem Modell das oben erwähnte Amiloridderivat Ethylisopropylamilorid.

In der US-Patentschrift 5 091 394 (HOE 89/F 288) sind Benzoylguanidine beschrieben, die in der dem Rest R(1) entsprechenden Stellung ein Wasserstoff-Atom tragen. Aus der EP-A-0 556 674 (HOE 92/F 034) sind Benzoylguanidine bekannt, in welchen aber die Substituenten nicht die nach der vorliegenden Erfindung beanspruchten Bedeutungen haben.

Im US-Patent 3 780 027 werden Acylguanidine beansprucht, die strukturell den Verbindungen der Formel I ähnlich sind und sich von im Handel befindlichen Schleifendiuretika, wie Bumetanid, ableiten. Entsprechend wird für diese Verbindungen eine starke salidiuretische Wirksamkeit berichtet.

Die erfindungsgemäßen Verbindungen zeichnen sich gegenüber den bekannten Benzoylguanidinen, z.B. aus der US -PS 5 091 394 (HOE 89/F 288) oder der Europäischen Offenlegungsschrift 602 522 (HOE 92/F 404) dadurch aus, daß der neuartige Substituent R(6)-C(OH)₂- aufgrund der geminalen Bis-hydroxy-Funktionalität eine sehr hohe Wasserlöslichkeit der Verbindungen I bewirkt.

Es war überraschend, daß die erfindungsgemäßen Verbindungen keine unerwünschten und nachteiligen salidiuretischen, jedoch sehr gute antiarrhythmische Eigenschaften aufweisen, wie sie beispielsweise für die Behandlung von Krankheitenwichtig sind, die bei Sauerstoff-Mangelerscheinungen auftreten. Die Verbindungen sind infolge ihrer pharmakologischen Eigenschaften als antiarrhythmische Arzneimittel mit cardioprotektiver Komponente zur Infarkt-prophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel I infolge Inhibition des zellulären Na⁺/H⁺ Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind. Darüberhinaus eignen sich die erfindungsgemäßen Verbindungen der Formel I ebenfalls zur Behandlungen von Formen des Schocks, wie beispielweise des allergischen, cardiogenen, hypovolämischen und des bakteriellen Schocks.

Darüberhinaus zeichnen sich die erfindungsgemäßen Verbindungen der Formel I durch starke inhibierende Wirkung auf die Proliferationen von Zellen, beispielsweise der Fibroblasten- Zellproliferation und der Proliferation der glatten Gefäßmuskelzellen, aus. Deshalb kommen die Verbindungen der Formel I als wertvolle Therapeutika für Krankheiten infrage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters (Na⁺/H⁺-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindungen der Formel I für die präventive Therapie zur Verhinderung der Genese des Bluthochdrucks, beispielweise der essentiellen Hypertonie, geeignet.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittel, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht,Alter, Gewicht und individueller Ansprechbarkeit des zu behandelnden Säugers.
Im Durchschnitt beträgt die tägliche Dosis einer Verbindung der Formel I bei einem etwa 75 kg schweren Patienten mindestens 0,001 mg/kg, vorzugsweise 0,01 mg/kg, bis höchstens 10 mg/kg, vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z.B. bis zu 4 Einzeldosen pro Tag. Insbesondere bei i.v. Anwendung, etwa bei einem Infarktpatienten auf der Intensivstation können bis zu 200 mg pro Tag notwendig werden.

### Liste der Abkürzungen:

- MeOH: Methanol
- DMF: N,N-Dimethylformamid
- NBS: N-Bromsuccinimid
- AIBN: α,α-Azo-bis-isobutyronitril
- EI: electron impact
- DCI: Desorption-Chemical Ionisation
- RT: Raumtemperatur
- EE: Ethylacetat (EtOAc)
- DIP: Diisopropylether
- MTB: Methyltertiärbutylether
- mp: Schmelzpunkt
- HEP: n-Heptan
- DME: Dimethoxyethan
- FAB: Fast Atom Bombardment
- CH₂Cl₂: Dichlormethan
- THF: Tetrahydrofuran
- eq: Äquivalent
- ES: Elektrospray-Ionisation

### Experimenteller Teil

### Allgemeine Vorschrift zur Herstellung von Benzoyl-guanidinen (I)

### Variante A: aus Benzoesäuren (II, L = OH)

0,01 M des Benzoesäurederivates der Formel II löst bzw. suspendiert man in 60 ml wasserfreiem THF und versetzt sodann mit 1,78 g (0,011 M) Carbonyldiimidazol. Nach dem Rühren über 2 Stunden bei RT werden 2,95 g (0,05 M) Guanidin in die Reaktionslösung eingetragen. Nach dem Rühren über Nacht destilliert man das THF unter vermindertem Druck (Rotavapor) ab, versetzt mit Wasser, stellt mit 2N HCL auf pH 6 bis 7 und filtriert das entsprechende Benzoylguanidin (Formel I) ab. Die so erhaltenen Benzoylguanidine können durch Behandeln mit wäßriger, methanolischer oder etherischer Salzsäure oder anderen pharmakologisch verträglichen Säuren in die entsprechenden Salze übergeführt werden.

### Allgemeine Vorschrift zur Herstellung von Benzoyl-guanidinen (I)

### Variante B: aus Benzoesäure-alkylestern (II, L = O-Alkyl)

5 mmol des Benzoesäure-alkylesters der Formel II sowie 25 mmol Guanidin (freie Base) werden in 15 ml Isopropanol gelöst oder in 15 ml THF suspendiert und bis zum vollständigen Umsatz (Dünnschichtkontrolle) unter Rückfluß gekocht (typische Reaktionszeit 2 bis 5 h). Das Lösungsmittel wird unter vermindertem Druck (Rotavapor) abdestilliert, in 300 ml EE aufgenommen und 3 x mit je 50 ml NaHCO₃-Lösung gewaschen. Es wird über Na₂SO₄ getrocknet, das Lösungsmittel im Vakuum abdestilliert und an Kieselgel mit einem geeigneten Laufmittel, z. B. EE/MeOH 5 : 1 chromatographiert. (Salzbildung vergleiche Variante A)

### Beispiel 1: 4-(1',1'-Bishydroxy-2',2',2'-trifluor)-ethyl-benzoylguanidin-hydrochlorid

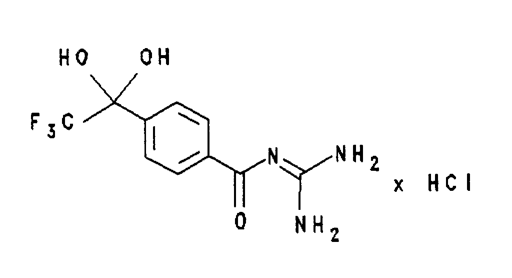

MS (ESI) M⁺+H: 278

### Beispiel 2: 4-Fluor-3-(1',1'-Bishydroxy-2',2',2'-trifluor)ethyl-benzoylguanidin-hydrochlorid

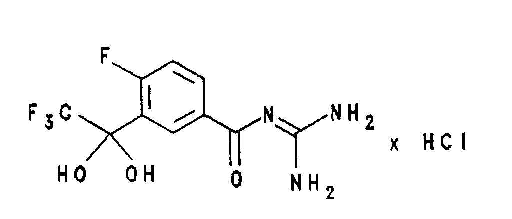

MS (ESI), M⁺+H: 296

### Pharmakologische Ergebnisse:

### Inhibition des Na⁺/H⁺-Exchangers:

| Beispiel | IC₅₀ (µmol/l) |
|---|---|
| 1 | 1.5 |

## Patentansprüche

1. Benzoylguanidine der Formel I 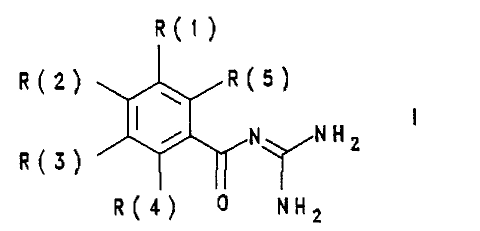 worin bedeuten:
mindestens einer der Substituenten R(1), R(2) und R(3)
R(6)-C(OH)₂-;
R(6) Perfluoralkyl mit 1, 2 oder 3-C-Atomen, das geradkettig oder verzweigt ist;
und die übrigen Substituenten R(1), R(2) und R(3)
unabhängig voneinander Wasserstoff, OH, F, Cl, Br, I, Alkyl mit 1, 2, 3, 4, 5 oder 6 C-Atomen, Cycloalkyl mit 3, 4, 5 oder 6 C-Atomen, Alkoxy mit 1, 2, 3 oder 4 C-Atomen oder Phenoxy,
das unsubstituiert ist oder substituiert ist mit 1 - 3 Substituenten ausgewählt auf der Gruppe bestehend aus F, Cl, Methyl und Methoxy;
oder
die übrigen Substituenten R(1), R(2) und R(3)
unabhängig voneinander Alkyl-SOₓ, -CR(7)=CR(8)R(9) oder -C≡CR(9);
x Null, 1 oder 2;
R(7) Wasserstoff oder Methyl;
R(8) und R(9)
unabhängig voneinander Wasserstoff, Alkyl mit 1, 2, 3 oder 4 C-Atomen, Cycloalkyl mit 3, 4, 5, 6, 7 oder 8 C-Atomen oder Phenyl,
das unsubstituiert ist oder substituiert ist mit 1 - 3 Substituenten ausgewählt auf der Gruppe bestehend aus F, Cl, CF₃, Methyl und Methoxy;
oder
die übrigen Substituenten R(1), R(2) und R(3)
unabhängig voneinander Phenyl, C₆H₅-(C₁-C₄)-Alkyl, Naphthyl, Biphenylyl, Chinolinyl, Isochinolinyl oder Imidazolyl,
wobei Chinolinyl, Isochinolinyl oder Imidazolyl über C oder N gebunden sind, und wobei Phenyl, C₆H₅-(C₁-C₄)-Alkyl, Naphthyl, Biphenylyl, Chinolinyl, Isochinolinyl und Imidazolyl unsubstituiert sind oder substituiert sind mit 1 - 3 Substituenten ausgewählt aus der Gruppe bestehend aus F, Cl, CF₃, CH₃, Methoxy, Hydroxy, Amino, Methylamino und Dimethylamino;
oder
die übrigen Substituenten R(1), R(2) und R(3)
unabhängig voneinander SR(10), -OR(10), -CR(10)R(11)R(12);
R(10)
-C_{f}H_{2f}-(C₃-C₈)-Cycloalkyl, Chinolinyl, Isochinolinyl, Pyridinyl, Imidazolyl oder Phenyl,
wobei die aromatischen Systeme Chinolinyl, Isochinolinyl, Pyridinyl, Imidazolyl und Phenyl unsubstituiert sind oder substituiert sind mit 1 - 3 Substituenten ausgewählt aus der gruppe bestehend aus F, Cl, CF₃, CH₃, Methoxy, Hydroxy, Amino, Methylamino und Dimethylamino;
f Null, 1 oder 2;
R(11) und R(12)
unabhängig voneinander wie R(10) definiert, Wasserstoff oder Alkyl mit 1, 2, 3 oder 4 C-Atomen;
R(4) und R(5)
unabhängig voneinander Wasserstoff, Alkyl mit 1, 2 oder 3-C-Atomen, F, Cl, Br, I, CN, OR(13), NR(14)R(15), -(CH₂)ₙ-(CF₂)ₒ-CF₃;
R(13), R(14) und R(15)
unabhängig voneinander Wasserstoff oder Alkyl mit 1, 2, 3 oder 4 C-Atomen;
n Null oder 1;
o Null, 1 oder 2;
sowie deren pharmakologisch akzeptable Salze.

2. Verbindung der Formel I nach Anspruch 1, dadurch gekennzeichnet, daß darin bedeuten:
mindestens einer der Substituenten R(1), R(2) und R(3)
CF₃-C(OH)₂-;
und die übrigen Substituenten R(1), R(2) und R(3)
unabhängig voneinander Wasserstoff, F, Cl, Alkyl mit 1, 2, 3, 4, 5 oder 6 C-Atomen, Cycloalkyl mit 3, 4, 5 oder 6 C-Atomen, Alkoxy mit 1, 2, 3 oder 4 C-Atomen oder Phenoxy,
das unsubstituiert ist oder substituiert ist mit 1 - 3 Substituenten ausgewählt auf der Gruppe bestehend aus F, Cl, Methyl und Methoxy;
oder
die übrigen Substituenten R(1), R(2) und R(3)
unabhängig voneinander Alkyl-SOₓ, -CR(7)=CR(8)R(9) oder -C≡CR(9);
x Null, 1 oder 2;
R(7) Wasserstoff oder Methyl;
R(8) und R(9)
unabhängig voneinander Wasserstoff, Alkyl mit 1, 2, 3 oder 4 C-Atomen, Cycloalkyl mit 3, 4, 5, 6, 7 oder 8 C-Atomen oder Phenyl,
das unsubstituiert ist oder substituiert ist mit 1 - 3 Substituenten ausgewählt auf der Gruppe bestehend aus F, Cl, CF₃, Methyl und Methoxy;
oder
die übrigen Substituenten R(1), R(2) und R(3)
unabhängig voneinander Phenyl, C₆H₅-(C₁-C₂)-Alkyl, Naphthyl, Biphenylyl, Chinolinyl, Isochinolinyl oder Imidazolyl,
wobei Chinolinyl, Isochinolinyl oder Imidazolyl über C oder N gebunden sind, und wobei Phenyl, C₆H₅-(C₁-C₄)-Alkyl, Naphthyl, Biphenylyl, Chinolinyl, Isochinolinyl und Imidazolyl unsubstituiert sind oder substituiert sind mit 1 - 3 Substituenten ausgewählt aus der Gruppe bestehend aus F, Cl, CF₃, CH₃, Methoxy, Hydroxy, Amino, Methylamino und Dimethylamino;
oder
die übrigen Substituenten R(1), R(2) und R(3)
unabhängig voneinander SR(10), -OR(10), -CR(10)R(11)R(12);
R(10)
-C_{f}H_{2f}-(C₃-C₈)-Cycloalkyl, Chinolinyl, Isochinolinyl, Pyridinyl, Imidazolyl oder Phenyl,
wobei die aromatischen Systeme Chinolinyl, Isochinolinyl, Pyridinyl, Imidazolyl und Phenyl unsubstituiert sind oder substituiert sind mit 1 - 3 Substituenten ausgewählt aus der gruppe bestehend aus F, Cl, CF₃, CH₃, Methoxy, Hydroxy, Amino, Methylamino und Dimethylamino;
f Null oder 1;
R(11) und R(12)
unabhängig voneinander wie R(10) definiert, Wasserstoff oder Alkyl mit 1, 2, 3 oder 4 C-Atomen;
R(4) und R(5)
unabhängig voneinander Wasserstoff, Alkyl mit 1, 2 oder 3-C-Atomen, F, Cl, OH, Methoxy, NH₂, -(CF₂)ₒ-CF₃;
o Null oder 1.

3. Verbindung der Formel I nach Anspruch 1, in der bedeuten:
R(1) CF₃-C(OH)₂-;
R(2) und R(3)
unabhängig voneinander Wasserstoff, F, Cl, Alkyl mit 1, 2, 3 oder 4 C-Atomen, Cycloalkyl mit 3, 4, 5 oder 6 C-Atomen, Alkoxy mit 1, 2, 3 oder 4 C-Atomen oder Phenoxy,
das unsubstituiert ist oder substituiert ist mit 1 - 3 Substituenten ausgewählt auf der Gruppe bestehend aus F, Cl, Methyl und Methoxy;
oder
R(2) und R(3)
unabhängig voneinander -CR(7)=CR(8)R(9) oder -C≡CR(9);
R(7) Wasserstoff oder Methyl;
R(8) und R(9)
unabhängig voneinander Wasserstoff, Alkyl mit 1, 2, 3 oder 4 C-Atomen, Cycloalkyl mit 3, 4, 5, 6, 7 oder 8 C-Atomen oder Phenyl,
das unsubstituiert ist oder substituiert ist mit 1 - 3 Substituenten ausgewählt auf der Gruppe bestehend aus F, Cl, CF₃, Methyl und Methoxy;
oder
R(2) und R(3)
unabhängig voneinander Phenyl, C₆H₅-(C₁-C₂)-Alkyl, Naphthyl, Biphenylyl, Chinolinyl, Isochinolinyl oder Imidazolyl,
wobei Chinolinyl, Isochinolinyl oder Imidazolyl über C oder N gebunden sind, und wobei Phenyl, C₆H₅-(C₁-C₄)-Alkyl, Naphthyl, Biphenylyl, Chinolinyl, Isochinolinyl und Imidazolyl unsubstituiert sind oder substituiert sind mit 1 - 3 Substituenten ausgewählt aus der Gruppe bestehend aus F, Cl, CF₃, CH₃, Methoxy, Hydroxy, Amino, Methylamino und Dimethylamino;
oder
R(2) und R(3)
unabhängig voneinander SR(10), -OR(10), -CR(10)R(11)R(12);
R(10)
-C_{f}H_{2f}-(C₃-C₈)-Cycloalkyl, Chinolinyl, Isochinolinyl, Pyridinyl, Imidazolyl oder Phenyl,
wobei die aromatischen Systeme Chinolinyl, Isochinolinyl, Pyridinyl, Imidazolyl und Phenyl unsubstituiert sind oder substituiert sind mit 1 - 3 Substituenten ausgewählt aus der gruppe bestehend aus F, Cl, CF₃, CH₃, Methoxy, Hydroxy, Amino, Methylamino und Dimethylamino;
f Null oder 1;
R(11) und R(12)
unabhängig voneinander wie R(10) definiert, Wasserstoff oder Alkyl mit 1, 2, 3 oder 4 C-Atomen;
R(4) und R(5)
unabhängig voneinander Wasserstoff, Alkyl mit 1, 2 oder 3-C-Atomen, F, Cl, OCH₃, -CF₃;
sowie deren pharmakologisch akzeptable Salze.

4. Verfahren zur Herstellung einer Verbindung I nach Anspruch 1, dadurch gekennzeichnet, daß man
eine Verbindung der Formel II 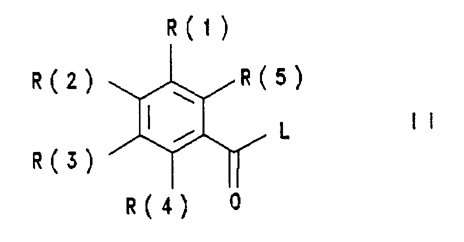 mit Guanidin umsetzt, worin R(1) bis R(5) die angegebene Bedeutung besitzen und L für eine leicht nucleophil substituierbare leaving group steht.

5. Verwendung einer Verbindung I nach einem oder mehreren der Ansprüche 1 bis 3 zur Herstellung eines Medikaments zu Behandlung von Arrhythmien.

6. Verwendung einer Verbindung I nach einem oder mehreren der Ansprüche 1 bis 3 zur Herstellung eines Medikaments zur Behandlung oder Prophylaxe des Herzinfarkts.

7. Verwendung einer Verbindung I nach einem oder mehreren der Ansprüche 1 bis 3 zur Herstellung eines Medikaments zur Behandlung oder Prophylaxe der Angina Pectoris.

8. Verwendung einer Verbindung I nach einem oder mehreren der Ansprüche 1 bis 3 zur Herstellung eines Medikaments zur Behandlung oder Prophylaxe von ischämischen Zuständen des Herzens.

9. Verwendung einer Verbindung I nach einem oder mehreren der Ansprüche 1 bis 3 zur Herstellung eines Medikaments zur Behandlung oder Prophylaxe von ischämischen Zuständen des peripheren und zentralen Nervensystems und des Schlaganfalls.

10. Verwendung einer Verbindung I nach einem oder mehreren der Ansprüche 1 bis 3 zur Herstellung eines Medikaments zur Behandlung oder Prophylaxe von ischämischen Zuständen peripherer Organe und Gliedmaßen.

11. Verwendung einer Verbindung I nach einem oder mehreren der Ansprüche 1 bis 3 zur Herstellung eines Medikaments zur Behandlung von Schockzuständen.

12. Verwendung einer Verbindung I nach einem oder mehreren der Ansprüche 1 bis 3 zur Herstellung eines Medikaments zum Einsatz bei chirurgischen Operationen und Organtransplantationen.

13. Verwendung einer Verbindung I nach einem oder mehreren der Ansprüche 1 bis 3 Herstellung eines Medikaments zur Konservierung und Lagerung von Transplantaten für chirurgische Maßnahmen.

14. Verwendung einer Verbindung I nach einem oder mehreren der Ansprüche 1 bis 3 zur Herstellung eines Medikaments zur Behandlung von Krankheiten, bei denen die Zellproliferation eine primäre oder sekundäre Ursache darstellt, und somit ihre Verwendung als Antiatherosklerotika, Mittel gegen diabetische Spätkomplikationen, Krebserkrankungen, fibrotische Erkrankungen wie Lungenfibrose, Leberfibrose oder Nierenfibrose, Prostatahyperplasie.

15. Verwendung einer Verbindung I nach einem oder mehreren der Ansprüche 1 bis 3 zur Herstellung eines wissenschaftliches Tools zur Inhibition des Na⁺/H⁺-Exchangers, zur Diagnose der Hypertonie und proliferativer Erkrankungen.

16. Heilmittel, enthaltend eine wirksame Menge einer Verbindung I nach einem oder mehreren der Ansprüche 1 bis 3.
